# EUROPÄISCHE PATENTSCHRIFT

(11) **EP 1 157 627 B1**
(45) Veröffentlichungstag und Bekanntmachung des Hinweises auf die Patenterteilung: **09.07.2003**
(21) Anmeldenummer: 01110905.5
(22) Anmeldetag: 05.05.2001
(51) Int. Cl.: A45D 29/05, B24D 7/10, B24D 17/00, A61B 17/54

(54) **Kühlbares rotierendes Schleifinstrument für die Fusspflege**
Rotating abrasive device for chiropody having cooling means
Instrument abrasif rotatif pour pédicure avec moyens de refroidissement

(30) Priorität: 13.05.2000 DE 20008683 U
(43) Veröffentlichungstag der Anmeldung: 28.11.2001
(73) Patentinhaber: Busch & Co.KG, 51766 Engelskirchen (DE)
(72) Erfinder: Busch, Gert, 51766 Engelskirchen (DE)
(74) Vertreter: Patentanwälte Maxton & Langmaack

(56) Entgegenhaltungen:
- EP-A- 0 530 536
- DE-A- 3 740 902
- DE-U- 8 600 670
- US-A- 1 820 581

## Beschreibung

Im Bereich der medizinischen Fußpflege ist es erforderlich, starke Verdickungen der Hornhaut, sogenannte Hyperkeratosen, abzutragen. Dies geschieht beispielsweise mit Hilfe von rotierenden Schleifinstrumenten, die eine hohe Abtragsleistung bieten. Die Behandlung erfolgte bisher mit sogenannten Kappenschleifern, die aus zwei Teilen bestehen, nämlich einem, mit einem Spannschaft für einen Antriebsmotor versehenen Gummiformkörper, auf den eine sogenannte Schleifkappe aufsetzbar ist. Die Schleifkappe besteht aus einem textilverstärkten Trägermaterial und ist auf ihrer Außenseite mit einem Schleifkorn aus Siliciumcarbid oder Edelkorund belegt. Die Schleifkappe wird als Einmalprodukt verwendet und muß dementsprechend bevorratet werden.

Dieses rotierende Instrument bietet durch seine Belegung mit grobem Siliciumcarbid oder Edelkorundkorn eine hohe Abtragsleistung, hat jedoch den Nachteil, daß nur in der sogenannten Trockentechnik gearbeitet werden kann, weil wegen des Materials der Schleifkappe ein Kühlmittel nicht eingesetzt werden kann und dementsprechend wegen der erheblichen Wärmeentwicklung sehr vorsichtig gearbeitet werden muß. Die Rundlaufgenauigkeit ist ebenfalls unbefriedigend, so daß insgesamt sowohl durch die Wärmeentwicklung als auch durch die infolge der mangelnden Rundlaufgenauigkeit entstehenden Vibrationen die Behandlung wenig patientenfreundlich ist.

Um die während der Behandlung entstehende Wärme durch Wasserkühlung abführen zu können, wurde ein zweiteiliger Ganzmetall-Schleifinstrument entwickelt, das einen Spannschaft und einen damit verbundenen Schleifkopf aufweist, der auf seiner Außenfläche mit Diamantkorn belegt ist. Der als Kappe ausgebildete Schleifkopf umschließt einen zum Spannschaft hin offenen Hohlraum und weist auf seiner Umfangsfläche radial ausgerichtete Bohrungen auf, so daß von der Einspannung des Spannschaftes her zugeführtes Wasser axial in den Hohlraum eintreten und über die radialen Bohrungen aus dem Hohlraum austreten kann. Die Kühlwirkung ist nur ungenügend und die zweiteilige Konstruktion ist nachteilig in bezug auf die Rundlaufgenauigkeit. Der Außendurchmesser des Schleifkopfes beträgt üblicherweise 10 mm und der Durchmesser des Spannschaftes 2,35 mm, so daß die Biegewechsellastbeanspruchung im Übergangsbereich zwischen Spannschaft und Schleifkopf des von Hand geführten Schleifinstrumentes die Verbindung überbeanspruchen kann und sich der Schleifkopf vom Spannschaft lösen kann.

Ein Beispiel des Stands der Technick ist in DE-A-3740902 gegeben.

Der Erfindung liegt die Aufgabe zugrunde, ein kühlbares rotierendes Schleifinstrument für die Fußpflege zu schaffen, bei dem die vorstehend beschriebenen Nachteile vermieden werden und das eine bessere Kühlwirkung erlaubt.

Diese Aufgabe wird gemäß der Erfindung gelöst durch ein kühlbares rotierendes Schleifinstrument für die Fußpflege, mit einem auf seiner Außenfläche mit Schleifkörnungen belegten schalenförmigen Schleifkopf, der mit einem Spannschaft verbunden ist und der einen zum Spannschaft hin offenen Hohlraum umschließt, der wenigstens eine radial nach außen gerichtete, die Außenfläche durchsetzende schlitzförmige Durchtrittsöffnung aufweist. Durch die Anordnung wenigstens einer, vorzugsweise von zwei oder drei schlitzförmigen Durchtrittsöffnungen ist ein wesentlich größerer Durchsatz von Kühlflüssigkeit aus dem Hohlraum auf die zu behandelnde Hautfläche möglich, so daß hier zum einen der Schleifkopf selbst, zum anderen aber auch die beschliffene Hornhautfläche besser gekühlt wird. Trotz der Anordnung der schlitzförmigen Durchtrittsöffnungen steht eine genügend große, mit Schleifkörnungen, insbesondere Diamantkörnungen belegte Schleiffläche zur Verfügung. Als Schleifkörnungen, außer Diamantkörnungen, kommen auch andere natürliche oder künstliche Schleifstoffe in Betracht. Aufgrund des größeren Durchtrittsquerschnittes einer schlitzförmigen Durchtrittsöffnung ergibt sich auch eine größere Spülwirkung, so daß die Durchtrittsöffnung sich nicht mit Hornhautabtrag zusetzen kann. Ein Vorteil dieses vollständig aus Metall hergestellten Schleifinstrumentes besteht darin, daß es gut sterilisierbar und/oder desinfizierbar ist und damit entsprechend auch mehrfach und somit kostensparend einsetzbar ist.

Die Außenfläche des Schleifkopfes kann unterschiedlich geformt sein. Neben einer zylindrischen Form mit angerundeter oder flacher Stirnfläche kommen beispielsweise auch eine Kegelform oder Formen mit gewölbter Außenflächen in Betracht.

In besonders vorteilhafter Ausgestaltung der Erfindung ist vorgesehen, daß die schlitzförmige Durchtrittsöffnung in axialer Richtung unter einem Winkel zur Achse des Spannschaftes geneigt ausgerichtet ist. Hierdurch ist gewährleistet, daß praktisch immer ein Teil der mit Diamantkorn belegten Umfangsfläche auf der abzutragenden Hornhautschicht anliegt, so daß ein "Springen" des Schleifkopfes, wie es bei einem axial ausgerichteten Schlitz möglich ist, vollständig vermieden ist.

In zweckmäßiger Ausgestaltung ist vorgesehen, daß der Hohlraum mit wenigstens einem axial ausgerichteten stirnseitig am Schleifkopf ausmündenden Durchtrittskanal versehen ist. Damit ist gewährleistet, daß auch bei einem Arbeiten mit der ebenfalls mit Diamantkorn belegten Stirnfläche des Schleifkopfes eine Kühlung der bearbeiteten Hornhautfläche erfolgt. Zweckmäßig sind auch hier wenigstens zwei - bezogen auf die Drehachse - diametral gegenüberliegende Durchtrittskanäle angeordnet, so daß der in der Regel mit Drehzahlen zwischen 5.000 und 15.000 Umdrehungen/min rotierende Schleifkopf wuchtfrei ist.

Zur Gewährleistung der Rundlaufgenauigkeit und zur Erhöhung der Lebensdauer ist ferner vorgesehen, daß der Schleifkopf mit dem Spannschaft einstückig stoffschlüssig verbunden ist. Hierzu wird das Schleifinstrument aus einem Stück hergestellt, wobei der Hohlraum des Schleifkopfes mit seinem Übergangsbereich zum Spannschaft hin und schließlich der Spannschaft selbst in einem Arbeitsgang hergestellt werden können. Als Material wird bevorzugt rostfreier Stahl verwendet. Für die Belegung der Außenfläche wird eine sogenannte splittrige Schleifkörnung, beispielsweise eine Diamantkörnung bevorzugt, d. h. ein Schleifkörnung, die mit einer Vielzahl von Vorsprüngen versehen ist, so daß jedes Korn mehrere "Schneiden" bildet.

Die Erfindung wird anhand schematischer Zeichnungen von Ausführungsbeispielen näher erläutert. Es zeigen:
- Fig. 1: eine erste Ausführungsform mit radialen Schlitzen im Schnitt,
- Fig. 2: eine Ausführungsform mit zusätzlichen axialen Durchtrittskanälen,
- Fig. 3 und 4: unterschiedliche Formen für den Schleifkopf,
- Fig. 4: eine zylindrische Ausführungsform mit sich kreuzenden Schlitzen.

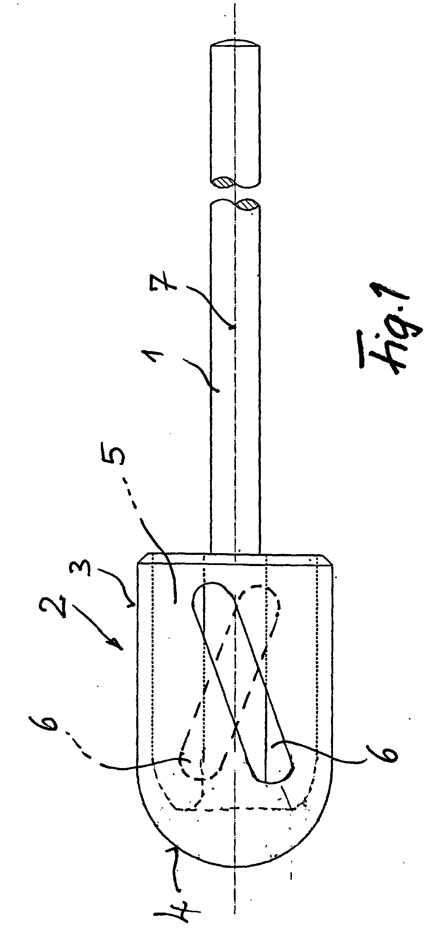

Das in Fig. 1 in großem Maßstab dargestellte Ganzmetall-Schleifinstrument besteht im wesentlichen aus einem Spannschaft 1, der mit einem schalenförmig ausgebildeten Schleifkopf 2 einstückig stoffschlüssig, d. h. aus einem Stück gefertigt, verbunden ist. Der Schleifkopf 2 weist eine zylindrische Umfangsfläche 3 auf, die an ihrem dem Spannschaft 1 abgekehrten Ende in eine gewölbte Stirnflächen 4 übergeht.

Die Außenfläche des Schleifkopfes mit seinen Flächenteilen 3 und 4 ist in üblicher Weise mit einer Diamantkörnung belegt.

Der schalenförmig ausgebildete Schleifkopf 2 umschließt einen Hohlraum 5, der zum Spannschaft 1 hin offen ist. Der Hohlraum 5 weist auf gegenüberliegenden Seiten zwei radial nach außen gerichtete, die zylindrische Umfangsfläche 3 durchsetzende schlitzförmige Durchtrittsöffnungen 6 auf. Die schlitzförmigen Durchtrittsöffnungen 6 sind in axialer Richtung unter einem Winkel zur Achse 7 des Spannschaftes 1 ausgerichtet, so daß die schlitzförmige Durchtrittsöffnung 6 je nach Drehrichtung des Schleifkopfes 2 "nachlaufend" oder "vorlaufend" ausgerichtet ist.

Das Schleifinstrument wird in ein Handstück mit Antrieb eingespannt und mit Drehzahlen zwischen 5.000 und 15.000 Umdrehung/min betrieben. Das Handstück ist an der Einspannstelle des Spannschaftes 1 mit Austrittsöffnungen für Kühlwasser versehen, das entlang des Spannschaftes 1 in den Hohlraum 5 eintritt und über die schlitzförmigen Durchtrittsöffnungen 6 auf die zu bearbeitende Hornhautfläche übertreten kann. Hierbei wird zum einen der Schleifkopf 2 von innen heraus selbst gekühlt und zum anderen auch die Wärmeentwicklung auf der zu bearbeitenden Hornhautfläche gemindert.

Die in Fig. 2 dargestellte Ausführungsform entspricht im wesentlichen der anhand von Fig. 1 beschriebenen Ausführungsform, wobei gleiche Bauteile mit gleichen Bezugszeichen versehen sind und dementsprechend auf die vorstehende Beschreibung verwiesen werden kann.

Bei der Ausführungsform gem. Fig. 2 ist der Hohlraum 5 zusätzlich mit zwei axial ausgerichteten und stirnseitig am Schleifkopf 2 ausmündenden Durchtrittskanälen 9 versehen, so daß auch die gewölbte Stirnflächen 4 mit Kühlwasser versorgt werden kann.

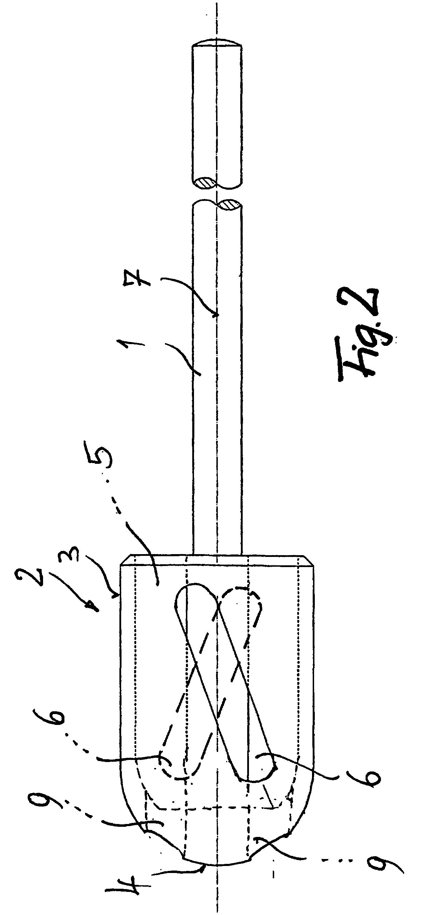

Fig. 3 und Fig. 4 zeigen beispielhaft weitere Ausführungsformen für den Schleifkopf 2. Zur Vereinfachung der Darstellung sind hier die schlitzförmigen Durchtrittsöffnungen 6 nicht gezeigt, da nur geometrische Gestaltungen der Außenfläche des Schleifkopfes gezeigt werden sollen.

Fig. 3 zeigt einen Schleifkopf mit einer kegelförmigen Umfangsfläche 3.1, die in eine abgerundete Stirnfläche 4.1 übergeht.

Fig. 4 zeigt einen Schleifkopf mit einer insgesamt gewölbten Außenfläche.

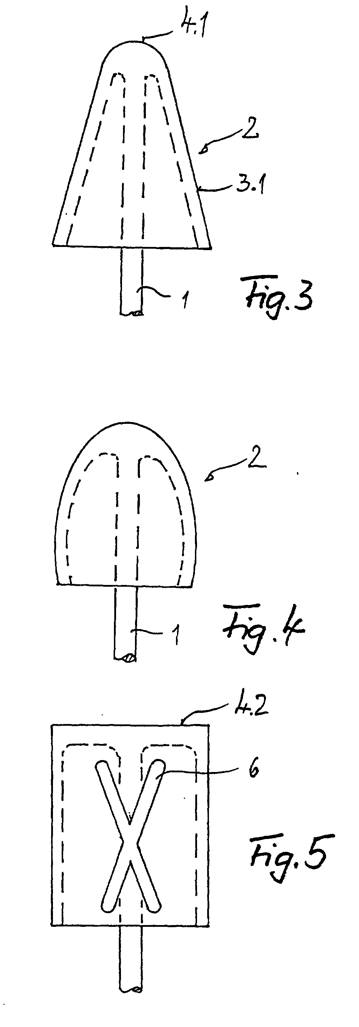

Fig. 5 zeigt einen i wesentlichen zylindrischen Schleifkopf mit ebener Stirnfläche 4.2. Der Vergleich mit Fig. 1 oder 2 läßt erkennen, daß die Wölbung der Stirnfläche praktisch beliebig gestaltet und somit optimal für den Einsatzzweck gestaltet werden kann. Auch bei dieser Ausführungsform können stirnseitig ausmündende Kanäle entsprechend den Kanälen 9 der Ausführungsform nach Fig. 2 vorgesehen werden.

Fig. 5 zeigt ferner eine kreuzweise Anordnung von schlitzförmigen Durchtrittsöffnungen 6. Es ist hierbei nicht erforderlich, die schlitzförmigen Durchtrittsöffnungen mehr oder weniger symmetrisch anzuordnen, wir in Fig. 5 dargestellt.

Durch die kreuzweise Anordnung der Schlitze wird eine zusätzliche, in Umfangsrichtung keilförmig ausgebildete Kante gebildet, die ebenfalls mit Schleifkörnung bestückt ist und damit eine besonders vorteilhafte "Schneidwirkung" aufweist.

Auch bei diesen oder ähnlichen Schleifkopfformen können neben den schlitzförmigen Durchtrittsöffnungen in den stirnseitigen Bereichen entsprechende stirnseitig austretende axiale Durchtrittskanäle 9 Kanäle vorgesehen sein.

## Patentansprüche

1. Kühlbares rotierendes Schleifinstrument aus Metall für die Fußpflege, mit einem auf seiner Außenfläche mit Schleifkörnungen belegten schalenförmigen Schleifkopf (2), der mit einem Spannschaft (1) verbunden ist und der einen zum Spannschaft (1) hin offenen Hohlraum (5) umschließt, der wenigstens eine radial nach außen gerichtete, die Außenfläche durchsetzende Durchtrittsöffnung (6) aufweist, **dadurch gekennzeichnet, daß** die Durchtrittsöffnung schlitzförmig ist.

2. Schleifinstrument nach Anspruch 1, **dadurch gekennzeichnet, daß** die Schleifkörnungen Diamantkörnungen sind.

3. Schleifinstrument nach Anspruch 1 oder 2, **dadurch gekennzeichnet, daß** die schlitzförmige Durchtrittsöffnung (6) in axialer Richtung unter einem Winkel zur Achse (7) des Spannschaftes (1) geneigt ausgerichtet ist.

4. Schleifinstrument nach einem der Ansprüche 1, 2 oder 3, **dadurch gekennzeichnet, daß** die schlitzförmigen Durchtrittsöffnungen zumindest zum teil sich kreuzend angeordnet sind.

5. Schleifinstrument nach Anspruch 1, 2 oder 4, **dadurch gekennzeichnet, daß** der Hohlraum (5) mit wenigstens einem axial ausgerichteten stirnseitig am Schleifkopf (2) ausmündenden Durchtrittskanal (9) versehen ist.

6. Schleifinstrument nach einem der Ansprüche 1 bis 5, **dadurch gekennzeichnet, daß** der Schleifkopf (2) mit dem Spannschaft (1) einstückig stoffschlüssig verbunden ist.

7. Schleifinstrument nach einem der Ansprüche 1 bis 6, **dadurch gekennzeichnet, daß** die Außenfläche mit einer splittrigen Schleifkörnung belegt ist.

8. Schleifinstrument nach einem der Ansprüche 1 bis 7, **dadurch gekennzeichnet, daß** es aus einem rostfreiem Stahl hergestellt ist.

## Claims

1. Coolable rotating abrasive instrument of metal for chiropody, with a shell-shaped abrasive head (2) which is coated on its outer surface with abrasive grit, is connected to a clamping shaft (1) and encompasses a cavity (5) which is open towards the clamping shaft (1) and comprises at least one through-opening (6) which is directed radially outwards and passes through the outer surface, **characterised in that** the through-opening is slot-shaped.

2. Abrasive instrument according to Claim 1, **characterised in that** the abrasive grit is diamond grit.

3. Abrasive instrument according to Claim 1 or 2, **characterised in that** the slot-shaped through-opening (6) is oriented in the axial direction at an angle to the axis (7) of the clamping shaft (1).

4. Abrasive instrument according to any one of Claims 1, 2 and 3, **characterised in that** the slot-shaped through-openings are disposed so as to intersect at least in part.

5. Abrasive instrument according to Claim 1, 2 or 4, **characterised in that** the cavity (5) is provided with at least one axially oriented through-channel (9) which opens out at the end side of the abrasive head (2).

6. Abrasive instrument according to any one of Claims 1 to 5, **characterised in that** the abrasive head (2) is integrally connected to the clamping shaft (1).

7. Abrasive instrument according to any one of Claims 1 to 6, **characterised in that** the outer surface is coated with angular abrasive grit.

8. Abrasive instrument according to any one of Claims 1 to 7, **characterised in that** it is manufactured from stainless steel.

## Revendications

1. Instrument abrasif rotatif refroidissable en métal pour les soins des pieds, comportant une tête abrasive (2) en forme de cuvette et garnie sur sa surface extérieure de granulations abrasives, qui est liée à une tige de serrage (1) et entoure une cavité (5) ouverte du côté de la tige de serrage (1), laquelle cavité contient au moins une ouverture de passage (6) orientée radialement vers l'extérieur et traversant la surface extérieure ; **caractérisé en ce que** ladite ouverture de passage est en forme de fente.

2. Instrument abrasif selon la revendication 1, **caractérisé en ce que** les granulations abrasives sont des granulations de diamant.

3. Instrument abrasif selon la revendication 1 ou 2, **caractérisé en ce que** l'axe de l'ouverture de passage en forme de fente (6) est orienté de manière inclinée sous un certain angle vis-à-vis de l'axe (7) de la tige se serrage (1).

4. Instrument abrasif selon l'une des revendications 1, 2 ou 3, **caractérisé par le fait que** les ouvertures de passage en forme de fente sont disposées, au moins en partie, de manière croisée.

5. Instrument abrasif selon la revendication 1, 2 ou 4, **caractérisé en ce que** la cavité (5) est pourvue d'au moins un conduit de passage (9) orienté axialement et débouchant à l'avant sur la tête abrasive (2).

6. Instrument abrasif selon l'une des revendications 1 à 5, **caractérisé en ce que** la tête abrasive (2) est liée à la tige de serrage (1) de manière jointive, de telle sorte qu'elles forment une seule pièce.

7. Instrument abrasif selon l'une des revendications 1 à 6, **caractérisé en ce que** la surface extérieure est garnie d'une granulation abrasive en éclats.

8. Instrument abrasif selon l'une des revendications 1 à 7, **caractérisé en ce qu'**il est fabriqué en acier inoxydable.
